# EUROPEAN PATENT APPLICATION

(11) **EP 3 492 457 A1**
(43) Date of publication of application: **05.06.2019**
(21) Application number: 17382826.0
(22) Date of filing: 01.12.2017
(51) Int. Cl.: C07D 311/16, C08J 3/24, C08J 3/28, C08G 18/32, C08L 75/04, C08L 75/12

(54) **COUMARIN DERIVATIVES, AND POLYOLS AND POLYURETHANES CONTAINING THEM**

(71) Applicant: Fundación Gaiker, 48170 Zamudio Vizcaya (ES); Consejo Superior De Investigaciones Científicas, 28006 Madrid (ES)
(72) Inventor: SEOANE RIVERO, Rubén, 48170 Zamudio- Bizkaia (ES); CUEVAS ZARRAGA, Jose María, 48170 Zamudio- Bizkaia (ES); NAVARRO CRESPO, Rodrigo, 28006 Madrid (ES); MARCOS FERNÁNDEZ, Angel Antonio, 28006 Madrid (ES)
(74) Representative: Balder IP Law, S.L.

(57) **Abstract**

The present invention relates to coumarin derivatives of formula (I), as well as to novel polyols and polyurethanes containing said compound of formula (I) which are photocrosslinkable, as well as their production and uses.

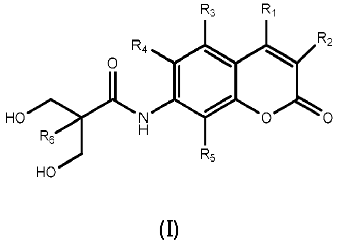

## Description

### FIELD OF THE INVENTION

The present invention relates to coumarin derivatives of formula (I), as well as to novel polyols and polyurethanes containing said compound of formula (I) which are photocrosslinkable, as well as their production and uses.

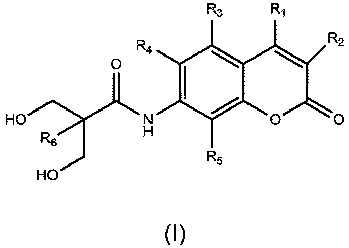

### BACKGROUND

It is known that reversible photocrosslinking and photo-cleavage ability is used for developing photo-response materials. Simple coumarins are oxygen heterocycles known as light-sensitive molecules able to act as photosensitive crosslinker to construct polymer networks. The capability of coumarin to reversibly photodimerize and photocleave under UV radiation is used to provide polymers with shape memory properties and self-healing properties. Polymers containing coumarin units perform effective photo-reactions with alternative UV radiation at different wavelengths by means of [2+2] photocycloaddition of couple of coumarin moieties.

In US 8,754,179 B2 there is disclosed a light activated shape memory cross-linked polymer bearing photo-reversible coumarin derivative, which is able to create a secondary photocrosslinked structure upon exposure to specific wavelength of light, where polymerizable end-group of coumarin derivative may be a mono-hydroxyl group.

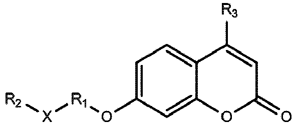

In CN1021535856 A there is disclosed a self-repairing reversible polyurethane film comprising phenolic hydroxyl coumarin derivative compounds as UV-sensitizers. In ES 2537618 A1 and WO2017/103295 A1 there are disclosed self-healing polyurethanes containing dihydroxy coumarins, polyols obtainable by reacting said dihydroxy coumarins and mixtures, where organic substituent in position C-7 of the coumarin may be an oxygen or methylene group. Preferably said ester diols were synthesized by protecting the hydroxyl groups of an acid, by activating the acid by anhydride formation and reacting the anhydride with the 7-hydroxycoumarin to yield the ester, which, after deprotection of the hydroxyl groups yields the coumarin diols.

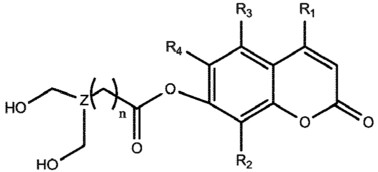

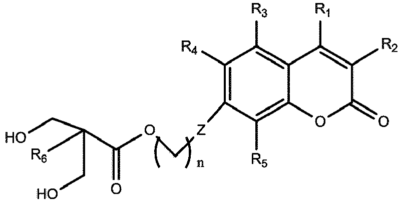

Ling et al. [Polymer 53 (2012) 2691-2698] disclose the use of dihydroxylated coumarin into the backbone of self-healing polyurethanes. Aguirresarobe et col. [Progress in Organic Coatings 99 (2016) 314-321] disclosed light responsive self-healing waterborne polyurethane dispersions with said coumarins.

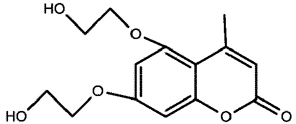

Seoane et col. [eXPRESS Polymer Letters Vol.10, No.2 (2016) 84-95; European Polymer Journal 76 (2016) 245-255; European Polymer Journal 92 (2017) 263-274] disclose improved dihydroxylated coumarin moieties, as disclosed in ES 2537618 A1 and WO2017/103295 A1, and a series of photo-crosslinkable branched and linear polyurethanes based on PCL with different content of said coumarin units. Salgado et col. [Macromolecular Materials and Engineer Vol. 302, Issue 4 (2017) 1600515; European Polymer Journal 93 (2017) 21-32] disclose photo-reversible polyurethanes and nanocomposites based on said coumarin diols.

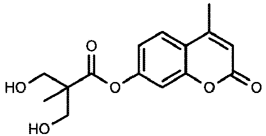

There is still a need of providing coumarin derivatives, which are useful for obtaining polyurethanes having self-healing properties, with better reactivity, in order to improve said self-healing properties, i.e. by a higher reactivity, e.g. improved dimerization kinetics, of the coumarin derivatives.

Further, the coumarin derivatives of the state of the art are sensitive to hydrolysis, and present problems of side-reactions during the preparation of polyols and polyurethanes, such as the transesterification of the terminal hydroxyl groups of alcohols with the ester group of the coumarin molecule; this type of side-reactions leads to the loss of the self-repairing capacity of the polymeric coating obtained from the coumarin derivatives.

### SUMMARY OF THE INVENTION

The present invention provides novel coumarin derivatives of formula (I), and polyols (A) and polyurethanes obtained from such derivatives, which overcome the problems of the prior art as outlined above.

Particularly, the derivatives of formula (I) according to the present invention have a better reactivity than known compounds, for example those disclosed in the prior art publication WO2017/103295A1; thus, the polyurethanes obtained from such derivatives, as the latter have improved dimerization kinetics, which provide the polyurethanes with improved self-healing properties.

Another advantage of the derivatives of formula (I) according to the present invention is that, contrary to what is needed when using other coumarin derivatives, e.g. those disclosed in the prior art publication WO2017/103295A1, there is no need for additional purification or crystallization steps at the end of the process of preparation of the derivatives of formula (I) according to the present invention, as both the final products and the products obtained in the intermediate steps are substantially pure. This is a significant advantage, as it reduces the time and cost of producing the compounds of formula (I) according to the invention, and thus those of producing polyols or polyurethanes obtained from such derivatives.

Therefore, according to a first aspect of the invention, there is provided a compound of formula (I): 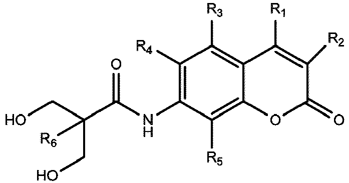 wherein
R₁, R₂, R₃, R₄, R₅ and R₆ are independently selected from the group consisting of H, alkyl and alkoxy C₁-C₆;
or a stereoisomer thereof.

In accordance with another aspect of the invention, there is provided a polyol (A), obtainable by reacting one or more of compounds of formula (I) with one or more compounds (B), in the presence of a catalyst, wherein compound (B) comprises:
a) a functional group selected from -C-(=O)-O- and -O-, and optionally at least one hydroxyl group, with the proviso that at least one hydroxyl group should be present when the -C-(=O)-O- and -O-groups are not part of a cycling compound; or
b) a functional group -O-CH(CH₃)-(C=O)-, and one or more hydroxyl groups.

In accordance with another aspect of the invention, there is provided a process for preparing a polyurethane by reaction of a mixture comprising:
- one or more polyisocyanates (C) which comprise two or more isocyanate groups, including without limitation aliphatic and aromatic isocyanates.
- one or more polyols, independently selected from one or more polyols (A) as defined above and one or more compounds of formula (I) as defined above, or mixtures thereof and,
- optionally, one or more polyols (D), comprising at least two hydroxyl groups, selected from the group consisting of polyester polyols, polyether polyols, aliphatic polyols, polycarbonate polyols, polyether-polycarbonate polyols and polyols of natural origin,
in an aprotic organic solvent,
in the presence of a catalyst,
wherein the ratio of equivalents of polyisocyanate (C) with respect to the sum of equivalents of polyol (A) and polyol (D) in the reaction mixture is between 2: 1 and 1: 1.2 and,
wherein the ratio of the sum of equivalents of polyol (A) and equivalents of compound of formula (I) with respect to equivalents of polyisocyanate (C) is from 1% to 95%.

According to another aspect of the invention, there is provided a method for preparing a polyurethane, comprising reacting a mixture that comprises:
- one or more polyisocyanates (C), which comprise at least two isocyanate groups;
- one or more polyols, independently selected from one or more polyols (A) as defined in any one of claims 4 to 7 and one or more compounds of formula (I) as defined in any one of claims 1 to 3, or mixtures thereof; and
- optionally, one or more polyols (D), which comprise at least two hydroxyl groups, selected from the group consisting of polyester polyols, polyether polyols, aliphatic polyols, polycarbonate polyols, polyether carbonate polyols and polyols of natural origin;
in an aprotic organic solvent;
in the presence of a catalyst;
wherein the ratio of equivalents of polyisocyanate (C) with respect to the sum of equivalents of polyol (A), equivalents of compound of formula (I) and equivalents of optional polyol (D) in the reaction mixture is comprised between 2:1 and 1:1.2; and
wherein the ratio of the sum of equivalents of polyol (A) and equivalents of compound of formula (I) with respect to the equivalents of polyisocyanate (C) is comprised between 1% and 95%.

According to another aspect of the invention, there is provided a method for preparing a polyurethane by reaction of a mixture comprising:
- one or more polyisocyanates (C), comprising two or more isocyanate groups;
- one or more polyols (D), comprising two or more hydroxyl groups, selected from the group consisting of polyester polyols, polyether polyols, aliphatic polyols, polycarbonate polyols, polyether-polycarbonate polyols and polyols of natural origin; and
- one or more compounds of formula (I) as defined above;
in an aprotic organic solvent,
in the presence of a catalyst,
wherein the ratio of equivalents of polyisocyanate (C) with respect to the sum of equivalents of polyol (A) and polyol (D) in the reaction mixture is between 2:1 and 1:1.2; and,
wherein the ratio between the sum of equivalents of polyol (A) and the equivalents of compound of formula (I) with respect to equivalents of polyisocyanate (C) is between 1% and 95%.

According to another aspect of the invention, there is provided a process for obtaining a polyurethane, comprising reacting a mixture that comprises:
- one or more polyisocyanates (C), comprising at least two isocyanate groups;
- one or more compounds of formula (I) as defined in any one of claims 1 to 3; and
- one or more polyols (D), comprising at least two hydroxyl groups, selected from the group consisting of polyester polyols, polyether polyols, allyl polyols, polycarbonate polyols, polyether carbonate polyols and polyols of natural origin;
in an aprotic organic solvent;
in the presence of a catalyst;
wherein the ratio of the equivalents of polyisocyanate (C) with respect to the sum of equivalents of polyol (D) and equivalents of compound of formula (I) in the reaction mixture is between 2:1 and 1:1.2; and
wherein the ratio between the sum of equivalents of polyol (D) and equivalents of compound of the formula (I) with respect to the equivalents of polyisocyanate (C) is comprised between 1% and 95%.

In accordance to another aspect of the invention, there is provided a polyurethane obtainable by the processes defined above.

According to another aspect of the invention, there is provided the use of above defined polyurethane, in the preparation of a coating.

In accordance to still another aspect of the invention, there is provided a method to repair the polyurethane defined above and or a coating that comprises said polyurethane, which comprises the steps of:
a) optionally, exposing the polyurethane or the coating comprising said polyurethane to light comprising radiation with a wavelength between 310 nm and 370 nm; and
b) exposing the polyurethane or the coating comprising said polyurethane to light comprising radiation with a wavelength between 200 and 260 nm.

### DETAILED DESCRIPTION OF THE INVENTION

### Definitions

In the frame of the present invention, the term "alkyl" refers to a straight or branched hydrocarbon chain radical, consisting of carbon and hydrogen atoms, which does not contain unsaturations, having 1 to 6 (i.e., 1, 2, 3, 4, 5 or 6), preferably 1 to 3 (i.e., 1, 2, or 3) carbon atoms, and which is attached to the rest of the molecule by a single bond; for example, but not limited to, methyl, ethyl, n-propyl, i-propyl, n-butyl, t-butyl, n-pentyl, etc.

The term "alkoxide", as used herein, refers to an alkyl radical, as defined above, attached to the rest of the molecule through an -O- group; for example, but not limited to, methoxyl, ethoxyl, n-propioxyl, isopropoxyl, n-butoxyl, t-butoxyl, etc.

The term "alkylene", as used herein, refers to a linear hydrocarbon chain radical, consisting of carbon and hydrogen atoms, which has the number of carbon atoms as indicated in each case, and which is attached to the remainder of the molecule by the two ends of the radical by simple bonds; for example, but not limited to, ethylene (-CH₂-CH₂-), n-propylene (-CH₂-CH₂-CH₂-), n-butylene (-CH₂-CH₂-CH₂-CH₂-), n-pentylene (-CH₂-CH₂-CH₂-CH₂-CH₂-), etc.

The term "cycloalkyl", as used herein, refers to a saturated carbocyclic ring, having from 3 to 8 carbon atoms (i.e., 3, 4, 5, 6, 7 or 8), preferably from 4 to 6 carbon atoms (i.e., 4, 5, or 6); for example, but not limited to, cyclopropyl, cyclobutyl, cyclopentyl, cyclohexyl, cycloheptyl and cyclooctyl.

The term "cycloalkylene", as used herein, refers to a saturated carbocyclic ring radical, having from 3 to 8 carbon atoms (i.e., 3, 4, 5, 6, 7, or 8), preferably from 4 to 6 carbon atoms (i.e., 4, 5 or 6), and which is attached to the remainder of the molecule through two different carbon atoms by simple bonds; for example, but not limited to, cyclopropylene, cyclobutylene, cyclopentylene, cyclohexylene, cycloheptylene and cyclooctylene.

The term "aryl", as used herein, refers to an aromatic hydrocarbon radical, such as phenyl, naphthyl or anthracyl. The aryl radical may be optionally substituted by one or more substituents, such as hydroxyl, halogen, alkyl, and alkoxide, as defined herein.

The term "halogen" or "halo", as used herein, refers to -F, -CI, -Br and -I.

The term "hydroxyl" or "hydroxy", as used herein, refers to an -OH group.

The term "aliphatic", as used herein, refers to cyclic or acyclic, linear or branched, saturated or unsaturated hydrocarbon compounds, excluding aromatics.

The term "aromatic", as used herein, refers to a mono- or polycyclic hydrocarbon comprising at least one unsaturated ring that satisfies the *Hückel* rule of aromaticity. Examples of aromatic rings are phenyl, indanyl, indenyl, naphthyl, phenethrile and anthracyl.

The term "stereoisomer", as used herein, refers to compounds formed by the same atoms, linked by the same sequence of bonds, but having different three-dimensional structures which are not interchangeable, for example isomers due to the presence of chiral centres (enantiomers, diastereomers, epimers and mixtures thereof, including the racemic mixture), or isomers due to the presence of multiple bonds (cis, trans and mixtures thereof).

The term "equivalent", in the present document, refers to the moles of compound per reactive units present in said compound, for example, one mole of diisocyanate corresponds to two equivalents of diisocyanate, whereas one mole of monoisocyanate corresponds to one equivalent of monoisocyanate.

The term "independently selected from", in the present document, means that the radicals following the terms may be the same or different. Any combinations are meant to be included.

### Compound of formula (I)

According to a first aspect, the present invention relates to a coumarin derivative, which is a compound of formula (I): 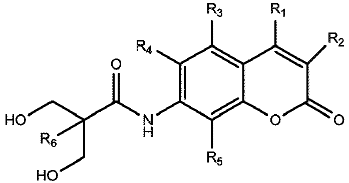 wherein
R₁, R₂, R₃, R₄, R₅ and R₆ are independently selected from the group consisting of H, alkyl and alkoxy C₁-C₆;
or a stereoisomer thereof.

For example, R₁, R₂, R₃, R₄, R₅ and R₆ may be independently selected from the group consisting of H, C₁-C₃ alkyl and C₁-C₃ alkoxide.

The present invention is directed, for example, to a compound of formula (I) as defined above, wherein R₁ is selected from the group consisting of H, C₁-C₃ alkyl and C₁-C₃ alkoxy; preferably, from the group consisting of H and C₁-C₃ alkyl; even more preferably, from the group consisting of H, methyl and ethyl; most preferably, R₁ is methyl; or to a compound of formula (I) as defined above, wherein R₂ is selected from the group consisting of H, C₁-C₃ alkyl and C₁-C₃ alkoxy; preferably, from the group consisting of H and C₁-C₃ alkyl; even more preferably, from the group consisting of H, methyl and ethyl; most preferably, R₂ is H.

The invention is also directed, for example, to a compound of formula (I) as defined above, wherein R₃ is selected from the group consisting of H, C₁-C₃ alkyl and C₁-C₃ alkoxy; preferably, from the group consisting of H and C₁-C₃ alkyl; even more preferably, from the group consisting of H, methyl and ethyl; most preferably, R₃ is H.

The invention is also directed, for example, to a compound of formula (I) as defined above, wherein R₄ is selected from the group consisting of H, C₁-C₃ alkyl and C₁-C₃ alkoxy; preferably, from the group consisting of H and C₁-C₃ alkyl; even more preferably, from the group consisting of H, methyl and ethyl; most preferably, R₄ is H.

The invention is also directed, for example, to a compound of formula (I) as defined above, wherein R₅ is selected from the group consisting of H, C₁-C₃ alkyl and C₁-C₃ alkoxy; preferably, from the group consisting of H and C₁-C₃ alkyl; even more preferably, from the group consisting of H, methyl and ethyl; most preferably, R₅ is H.

The invention is also directed, for example, to a compound of formula (I) as defined above, wherein R₆ is selected from the group consisting of H, C₁-C₃ alkyl and C₁-C₃ alkoxy; preferably, from the group consisting of H and C₁-C₃ alkyl; even more preferably, from the group consisting of H, methyl and ethyl; most preferably, R₆ is methyl.

Any combinations of possible definitions of R₁, R₂, R₃, R₄, R₅ and R₆ are intended to be included in the scope of the present invention.

The present invention is also directed, for example, to a compound of formula (I) as defined above, wherein R₁ and R₆ are independently selected from the group consisting of H, methyl and ethyl, and R₂, R₃, R₄ and R₅ are H.

The present invention is also directed, for example, to a compound of formula (I) as defined above, wherein R₁ and R₆ are methyl, and R₂, R₃ R₄ and R₅ are H.

The compound of formula (I) can be obtained by an amidation reaction between the acid (IV), or a corresponding precursor thereof, such as a corresponding anhydride or acid halide, and coumarin (III), as shown in Scheme 1, by methods known to the person skilled in the art and described by Gernigon et al [J. Org. Chem., 2012, 77, 8386-8400].

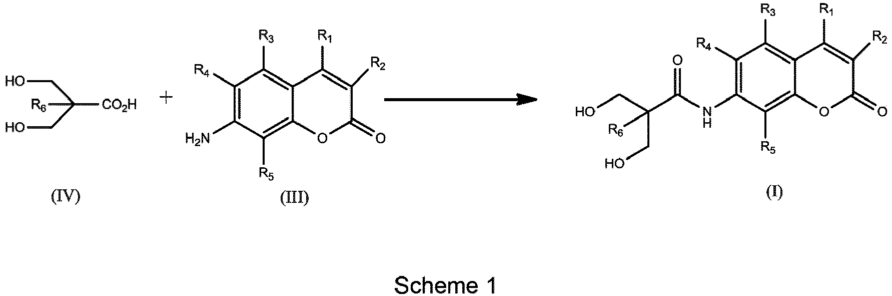

The alcohol groups of the acid (IV) may be protected by alcohol protecting groups (GP) known to the person skilled in the art and described, for example in Wuts, P.G.M. and Greene T.W., Protecting groups in Organic Synthesis, 4th Ed. Wiley-Interscience; and in Kocienski P.J., Protecting Groups, 3rd Ed. Georg Thieme Verlag, as shown in Scheme 2 below. For example, ethers (including acetal formation) and silylated derivatives.

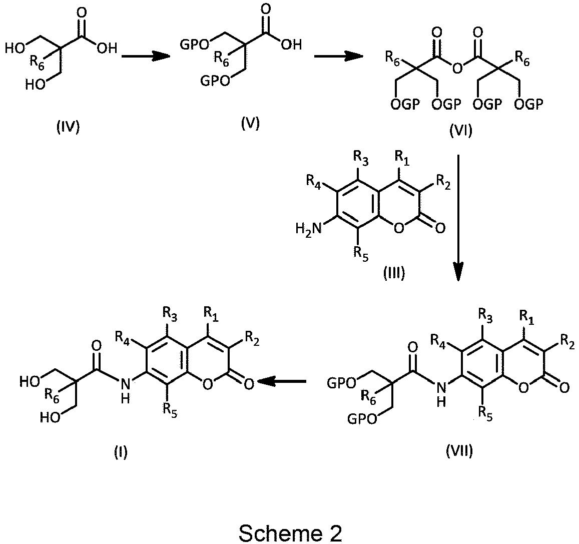

Preferably, the process for obtaining the compound of formula (I) comprises the steps defined in Scheme 2, that is, protection of the hydroxyl groups of the acid (IV) to yield the acid (V), by conventional methods, followed by activation of the acid (V) by forming the anhydride (VI) by conventional methods, as for example in the presence of dicyclohexylcarbodiimide (DDC), and reaction of the anhydride (VI) with the coumarin (III) to yield the amide (VII), which, after deprotection of the hydroxyl groups by hydrolysis, yields the compound of formula (I).

### Polyol (A)

According to a second aspect, the invention relates to a polyol (A), obtainable by the condensation reaction of one or more compounds of formula (I) as defined above, with one or more compounds (B), in the presence of a catalyst, wherein the compound (B) comprises:
a) a functional group, selected from -C(=O)-O- and -O- (which will participate in the condensation reaction, binding to the hydroxyl groups of compound of formula (I)), and optionally at least one hydroxyl group, with the proviso that, when the functional group selected from -C(=O)-O- and -O- is not part of a cycle, the said at least one hydroxyl group must be present; or
b) a functional group -O-CH(CH₃)-(C=O)- and at least one hydroxyl group.

Thus, the compound (B) may for example be cyclic compound, comprising a functional group selected from -C(=O)-O- and -O-, and no further hydroxyl group, or a cyclic compound comprising both a functional group selected from -C(=O)-O- and -O-, and at least one hydroxyl group. In the case the functional group selected from -C(=O)-O- and -O- is not part of a cycle, the said at least one hydroxyl group must be present.

Compound (B) may also be a lactone or a cyclic ether, i.e. an aliphatic heterocycle, comprising one or more -C(=O)-O- or -O- groups forming part of a cycle. Examples of this type of compounds are lactones, such as ε-caprolactone, v-butyrolactone, d-varelolactone, glycolide, lactide, etc. and their copolymers; and cyclic ethers, such as ethylene oxide and propylene oxide.

The compound (B) may also have a linear structure, comprising a -C(=O)-O- group or a -O- group (which will participate in the reaction, binding to one of the hydroxyl groups of compound of formula (I)), and at least one hydroxyl group. Examples of this type of compounds are hydroxy acids, which comprise a carboxylic acid at one end and a hydroxyl group at the other end, and the hydroxyesters, which comprise an ester at one end and a hydroxyl group at the other end.

Alternatively, compound (B) may comprise the functional group -O-CH(CH₃)-(C=O)-, which is present for example in lactic acid, and at least one hydroxyl group. Examples of this type of compounds are L-lactic acid, D-lactic acid, and its oligomers from L-lactic, D-lactic, or mixtures of both.

As indicated above, the compound (B) may be a lactone; the lactone is preferably selected from, but not limited to, the group consisting of ε-caprolactone, δ-valerolactone, γ-butyrolactone, and β-propiolactone; more preferably selected from the group consisting of ε-caprolactone and δ-valerolactone; most preferably the lactone is ε-caprolactone.

As indicated above, the polyol (A) is obtained by reaction of at least one compound of formula (I) and at least one compound (B). This means, that for example one compound of formula (I) may be reacted with one compound of formula (B), as they have been defined above. Alternatively, one compound of formula (I) may be reacted with two or more compounds (B); or two or more compounds of formula (I) may be reacted with one compound (B); or two or more compounds of formula (I) may be reacted with two or more compounds (B).

The catalyst used for the condensation reaction of one or more compounds of formula (I) as defined above, with one or more compounds (B) as defined above, may be a tertiary amine, such as, for example but not limited to, triethylamine, triethylenediamine, methylethanolamine, triethanolamine, dimethylethanolamine, pyridine, 1,4-diazabicyclo [2.2.2] octane, dimethylcyclohexylamine, dimethylpiperazine; organic derivatives of tin, mercury, lead, bismuth, zinc and potassium, such as for example, but not limited to, tin 2-ethyl-hexanoate (commonly called tin octanoate), tin isooctanoate, dibutyl tin dilaurate, potassium octanoate and potassium acetate. Preferably, the catalyst is selected from the group consisting of tin octanoate, tin isooctanoate, dibutyltin dilaurate, triethylamine, triethylenediamine, methylethanolamine, triethanolamine, dimethylethanolamine, pyridine, 1,4-diazabicyclo [2.2.2] octane, dimethylcyclohexylamine; more preferably, the catalyst is independently selected from the group consisting of tin octanoate, tin isooctanoate and dibutyltin dilaurate; most preferably, the catalyst is tin octanoate.

Any of the above defined compounds of formula (I) may be reacted with any of the above defined compounds (B); any of said combinations may in turn be combined with any of the possible catalysts indicated above.

The reaction may be carried out in the absence of solvent.

Alternatively, the reaction may be carried out in the presence of an aprotic organic solvent. Said solvent may be selected from the group consisting of dimethylformamide, butyl acetate, dimethyl sulfoxide, dimethylacetamide, tetrahydrofuran, dioxane, ethyl acetate, acetone, cyclohexanone, ethyl methyl ketone, acetonitrile, hexane, toluene, dichloromethane and mixtures thereof; more preferably, the solvent is independently selected from the group consisting of dimethylformamide, butyl acetate, dimethylacetamide, dimethyl sulfoxide and mixtures thereof; most preferably, the aprotic organic solvent is dimethylformamide, butyl acetate or a mixture thereof.

Any combinations of compounds of formula (I), compounds (B) and catalysts, as defined above, may be used in any of the indicated aprotic organic solvents.

Preferably, the reaction is carried out at a temperature between 50 °C and 150 °C, more preferably between 70 °C and 130 °C, most preferably between 90 °C and 110 °C.

The polyol (A), obtainable by reaction of one or more compounds (B) and one or more compounds of formula (I), may have a molar ratio of compounds (B) with respect to compounds of formula (I) of between 80:1 and 1:1, more preferably of between 40:1 and 1.5:1.

The polyol (A) may have, but not necessarily, an average molecular weight of 200 Dalton to 10000 Dalton, preferably of 200 Dalton to 2000 Dalton.

Polyol (A) may be a compound of formula (II): 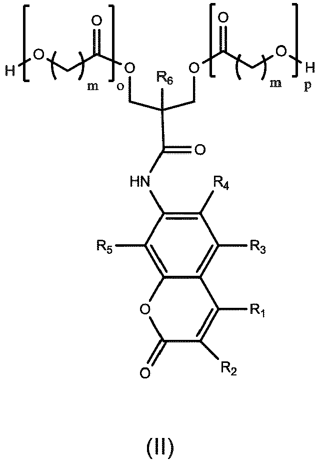 wherein R₁, R₂, R₃, R₄, R₅ and R₆ are as defined above for the compound of formula (I), o and p are independently selected from an integer between 0 and 40 (i.e., 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40), and m is selected from an integer between 2 and 10 (i.e. 2, 3, 4, 5, 6, 7, 8, 9 or 10), with the proviso that at least one of **o** and **p** is different from zero.

Preferably, **o** and **p** are independently selected from an integer between 0 and 20; or from an integer between 0 and 15; or from an integer between 0 and 10; or an integer between 2 and 8; or an integer between 3 and 5; preferably, one of **o** or **p** is 4, or both of **o** and **p** are 4.

Preferably, **m** is an integer between 3 and 7; or an integer between 4 and 6; preferably, **m** is 5.

A compound of formula (I), for example, be one wherein **o** and **p** are 4 and **m** is 5. Nevertheless, all possible combinations of **o** and **p** and **m** are intended to be included in the scope of the present invention.

### Polyurethane

Generally, polyurethanes are obtained from three monomers: a polyol having a relatively long and flexible chain, which constitutes the soft segments of the polyurethane; a polyisocyanate; and a short chain polyol, also referred to as a chain extender, in the case it is difunctional or crosslinking, if it has a functionality higher than 2. The reaction of the polyisocyanate with the chain extender forms the hard segments of polyurethane.

In a third aspect, the present invention relates to a process for obtaining a polyurethane, said method comprising reacting a mixture that comprises:
- one or more polyisocyanates (C), which comprise at least two isocyanate groups;
- one or more polyols, independently selected from one or more polyols (A) as defined above and one or more compounds of formula (I) as defined above, or mixtures thereof; and
- optionally, one or more polyols (D), comprising at least two hydroxyl groups, selected from the group consisting of polyester polyols, polyether polyols, aliphatic polyols, polycarbonate polyols, polyether carbonate polyols and polyols of natural origin;
in an aprotic organic solvent;
in the presence of a catalyst;
wherein the ratio of the equivalents of polyisocyanate (C) with respect to the sum of equivalents of polyol (A), equivalents of compound of formula (I) and equivalents of optional polyol (D) in the reaction mixture is between 2:1 and 1:1.2; and
wherein the ratio of the sum of equivalents of polyol (A), equivalents of optional polyols (D) and equivalents of compound of formula (I) with respect to the equivalents of polyisocyanate (C) is between 1% and 95%, preferably between 2% and 60%.

In the method of the third aspect, the mixture defined above may contain both the polyol (A) and the polyol (D). In this case, the one or more compounds of formula (I) may or not be present. In this embodiment, the ratio of the equivalents of polyisocyanate (C) with respect to the sum of equivalents of polyol (A), equivalents of compound of formula (I) (if present) and equivalents of polyol (D) in the reaction mixture is preferably between 2:1 and 1:1,2.

The method of the third aspect may comprise reacting the mixture defined above, wherein the polyols of the mixture do not comprise polyol (A) (i.e., compound of formula (I) and polyol (D) are present); alternatively, wherein the polyols of the mixture do not comprise the compound of formula (I) (i.e., polyol (A) and polyol (D) are present). In these embodiments, the ratio of equivalents of polyisocyanate (C) with respect to either the sum of equivalents of polyol (A) and equivalents of polyol (D), or the sum of equivalents of compound of formula (I) and equivalents of polyol (D) in the reaction mixture is preferably between 2:1 and 1:1.2.

In a fourth aspect, the invention relates to a process for obtaining a polyurethane, comprising reacting a mixture that comprises:
- one or more polyisocyanates (C), comprising at least two isocyanate groups;
- one or more polyols (D), comprising at least two hydroxyl groups, selected from the group consisting of polyester polyols, polyether polyols, aliphatic polyols, polycarbonate polyols, polyether carbonate polyols and polyols of natural origin; and
- at least one compound of formula (I) as defined above (which would act as a chain extender);
in an aprotic organic solvent;
in the presence of a catalyst;
wherein the ratio of the equivalents of polyisocyanate (C) with respect to the sum of equivalents of polyol (D) and equivalents of compound of formula (I) in the reaction mixture is between 2:1 and 1:1.2; and
wherein the ratio of the sum of equivalents of polyol (D) and equivalents of compound of formula (I) with respect to the equivalents of polyisocyanate (C) is between 1% and 95%.

The term "polyisocyanate", in the frame of the present invention, should be understood as a compound comprising two or more, preferably two, aliphatic and/or aromatic isocyanate groups, preferably aliphatic. Examples of aliphatic polyisocyanates are hexamethylene diisocyanate (HDI), 2,2,4-trimethylhexamethylene diisocyanate (TMDI), tetramethylene diisocyanate, octamethylene diisocyanate, decamethylene diisocyanate, dodecamethylene diisocyanate, tetradecamethylene diisocyanate, dimeryl diisocyanate (DDI), 1,1,6,6-tetrahydroperfluorohexamethylene diisocyanate (TFDI), isophorone diisocyanate (IPDI), 1,4-cyclohexane diisocyanate (CDI), 1,3-dicyclohexane diisocyanate, 1,2-dicyclohexane diisocyanate, 1,3-bis(isocyanatomethyl) cyclohexane (H₆XDI), 4,4'-dicyclohexylmethane diisocyanate (H₁₂MDI), and mixtures thereof. Examples of aromatic polyisocyanates are 2,4- and 2,6-toluene diisocyanate (TDI), para-phenylene diisocyanate (PPDI), 4,4'-diphenylmethane diisocyanate and its 2,4' and 2,2' isomers (MDI), tetramethylxylene diisocyanate (TMXDI), 1,5-naphthalene diisocyanate (NDI), 1,3-bis(1-isocyanate-1-methylethyl)benzene, meta-xylylene diisocyanate, and mixtures thereof.

More specifically, polyisocyanate (C) may be selected from the group consisting of straight or branched C₂-C₂₀ alkylene diisocyanate, optionally substituted with 1 to 10 (1, 2, 3, 4, 5, 6, 7, 8, 9, or 10) substituents, independently selected from halogen; C₃-C₈ cycloalkylene diisocyanate, optionally substituted with 1 to 4 substituents, independently selected from the group consisting of C₁-C₃ alkyl and halogen; C₁-C₆ alkylene cycloalkylene - C₃-C₈ cycloalkylene diisocyanate, optionally substituted with from 1 to 4 substituents, independently selected from the group consisting of C₁-C₃ alkyl and halogen; and C₃-C₈ cycloalkylene - C₁-C₆ alkylene - C₃-C₈ cycloalkylene diisocyanate, optionally substituted with 1 to 4 substituents, independently selected from the group consisting of C₁-C₃ alkyl and halogen Preferably, polyisocyanate (C) is selected from the group consisting of hexamethylene diisocyanate (HDI), 2,2,4-trimethylhexamethylene diisocyanate (TMDI), tetramethylene diisocyanate, octamethylene diisocyanate, decamethylene diisocyanate, dodecamethylene diisocyanate, tetradecamethylene diisocyanate, dimeryl diisocyanate (DDI), 1,1,6,6-tetrahydroperfluorohexamethylene diisocyanate (TFDI), isophorone diisocyanate (IPDI), 1,4-cyclohexane diisocyanate (CDI), 1,3-dicyclohexane diisocyanate, 1,2-dicyclohexane diisocyanate, 1,3-bis (isocyanatomethyl) cyclohexane (H6XDI), 4,4'-dicyclohexylmethane diisocyanate (H12MDI) and mixtures thereof More preferably, polyisocyanate (C) is selected from the group consisting of hexamethylene diisocyanate (HDI), 2,2,4-trimethylhexamethylene diisocyanate (TMDI), tetramethylene diisocyanate, octamethylene diisocyanate, decamethylene diisocyanate, dodecamethylene diisocyanate and tetradecamethylene diisocyanate. Most preferably, the polyisocyanate (C) is hexamethylene diisocyanate (HDI).

The polyol (D) comprising at least two hydroxyl groups, in the frame of the present invention, refers to a compound having at least two hydroxyl groups, and is selected from the group consisting of polyester polyols, polyether polyols, aliphatic polyols (these also include polybutadiene polyols, wherein the aliphatic fragment is a chain derived from the polymerization of one or more butadiene groups), polycarbonate polyols, polyether carbonate polyols and naturally occurring polyols such as those mentioned above. Preferably, polyol (D) is selected from the group consisting of polyester polyols, polyether polyols, aliphatic polyols (these also include the polybutadiene polyols, wherein the aliphatic fragment is a chain derived from the polymerization of one or more butadiene groups), polycarbonate polyols and polyether carbonate polyols. More preferably, it is selected from the group consisting of polyester polyols and polyether polyols.

Preferably, the polyols have an average molecular weight between 200 Dalton and 10000 Dalton, more preferably between 200 Dalton and 4000 Dalton; more preferably, said polyols are selected from the group consisting of polyester polyols and polyether polyols.

The aliphatic polyols comprise, as functional groups, only the terminal hydroxyl groups. Preferred aliphatic polyols are diols, such as 1,2-butanediol, 1,3-butanediol, 1,4-butanediol, 2,3-butanediol, 1,3-propanediol, 1,2-propanediol and mixtures of two or more thereof. The aliphatic polyols also include the polybutadienes, wherein the aliphatic fragment is one or more butadiene groups.

The polyether polyols comprise, in addition to the terminal hydroxyl groups, non-terminal ether groups. Polyether polyols include polyethylene glycols, polypropylene glycols, mixed polyglycols based on ethylene oxide and propylene oxide, polytetramethylene glycols and polytetrahydrofurans.

The polyester polyols comprise, in addition to the terminal hydroxyl groups, non-terminal ester groups. Polyester polyols are obtained by condensation of diols and dicarboxylic acids, their anhydrides and esters. Examples of polyester polyols are the condensation products based on ethylene glycol, 1,4-butylene glycol, 1,6-hexamethylene glycol or neopentyl glycol, with adipic acid or isophthalic acid. Polycaprolactones and (meth)acrylic polyols also belong to the group of polyol polyesters. The polycaprolactones are obtained by the reaction between phosgene, aliphatic carbonates or aromatic carbonates, such as diphenylcarbonate or diethylcarbonate, with dihydric or polyhydric alcohols. The polycaprolactones are obtained by polyaddition of lactones, such as, for example, ε-caprolactone, with a starter compound having reactive hydrogen atoms, such as water, alcohols, amines or bisphenol A. The (meth)acrylic polyols are obtained by radical copolymerization of
(a) monomers of (meth) acrylic acids or esters, such as acrylic acid, methyl acrylate, ethyl acrylate, propyl acrylate, butyl acrylate and hexyl acrylate, methacrylic acid, methyl methacrylate, ethyl methacrylate, propyl methacrylate, butyl methacrylate and hexyl methacrylate; and
(b) monomers of hydroxyalkyl (meth) acrylates, such as hydroxyethyl acrylates, hydroxyethyl acrylates, hydroxypropyl methacrylates, hydroxypropyl methacrylates, glycidyl acrylate and glycidyl methacrylate.

The polycarbonate polyols comprise, in addition to the hydroxyl groups, non-terminal carbonate groups. Examples of such polyols are hexanediol polycarbonate, butanediol polycarbonate, 1,2-propanediol polycarbonate and other diols, and mixtures of diols, such as Oximer® from Perstorp, Eternacoll® from UBE, or Desmophen® from Bayer.

Also combinations of polyester polyols, polycaprolactone polyols, (meth)acrylic polyols and polycarbonate polyols may be used.

The polyether carbonate polyols comprise, in addition to the terminal hydroxyl groups, carbonate groups and ether groups. Examples of polyether carbonate polyols are polyether carbonate polyols obtained from propylene oxide and carbon dioxide (CO₂) with an alternating or random structure, with different contents of carbonate groups in their structure, such as, for example, the Converge® of Novomer.

The polyols of natural origin are those that provide from natural sources, such as vegetable oils. The polyols may be directly extracted from said natural sources.

The examples include castor oil or polyols obtained by derivatization of oils such as soya, sunflower or canola.

The polyol (D) comprising at least two hydroxyl groups may be selected from the group consisting of polycaprolactones, polyethylene glycols, polypropylene glycols, mixed polyglycols of ethylene oxide and propylene oxide, polytetramethylene glycols, polytetrahydrofurans and mixtures thereof; polyol (D) may have an average molecular weight between 200 Dalton and 10000 Dalton, preferably between 200 Dalton and 10000 Dalton; preferably, polyol (D) is selected from the group consisting of polycaprolactones, polyethylene glycols, polypropylene glycols, mixed polyglycols of ethylene oxide and propylene oxide and mixtures thereof, wherein the polyol (D) has an average molecular weight between 200 Daltons and 10,000 Dalton; most preferably, polyol (D) is selected from the group consisting of polycaprolactones, polyethylene glycols and mixtures thereof, wherein the polyol (D) has an average molecular weight between 200 Dalton and 4000 Dalton.

"Aprotic organic solvent" in the frame of the present invention is preferably an organic solvent selected from the group consisting of dimethylformamide, butyl acetate, dimethylsulfoxide, dimethylacetamide, tetrahydrofuran, dioxane, ethyl acetate, acetone, cyclohexanone, ethyl methyl ketone, acetonitrile. hexane, toluene, dichloromethane and mixture thereof; preferably, it is selected from the group consisting of dimethylformamide, butyl acetate, dimethylacetamide, dimethyl sulfoxide and mixture thereof; most preferably, the aprotic organic solvent is dimethylformamide, butyl acetate or a mixture thereof.

The catalyst is a catalyst suitable for carrying out the polyurethane formation reaction, such as tertiary amines, such as, for example, triethylamine, triethylene diamine, methylethanolamine, triethanolamine, dimethylethanolamine, pyridine, 1,4-diazabicyclo [2.2.2] octane, dimethylcyclohexylamine, dimethylpiperazine; organic derivatives of tin, mercury, lead, bismuth, zinc or potassium, such as for example tin octanoate, tin isooctanoate, dibutyl tin dilaurate, potassium octanoate and potassium acetate.

For example, the catalyst is selected from the group consisting of tin octanoate, tin isooctanoate, dibutyltin dilaurate, triethylamine, triethylenediamine, methylethanolamine, triethanolamine, dimethylethanolamine, pyridine, 1,4-diazabicyclo [2.2.2] octane, dimethylcyclohexylamine and potassium octanoate; more preferably, it is selected from the group consisting of tin octanoate, tin isooctanoate, dibutyltin dilaurate and potassium octanoate; most preferably, the catalyst is tin octanoate.

Typically, the catalyst is added in an amount comprised between 0.01 and 5 mole%, with respect to the moles of polyisocyanate (C), preferably between 0.01 and 0.05 mole% with respect to the moles of polyisocyanate (C).

The process of forming polyurethane is preferably carried out at a temperature between 50 °C and 150 °C, more preferably between 50 °C and 100 °C, most preferably between 65 °C and 85 °C.

Typically, the formation of polyurethane is carried out by stirring during a time not exceeding 24 hours, preferably below 12 hours, followed by heating to a temperature higher than room temperature, and not higher than 150 °C, preferably lower than 100 °C, most preferably between room temperature and 85 °C.

The process according to the invention may comprise reacting a mixture comprising one or more polyisocyanates (C) and one or more polyols (A). Any combinations thereof are comprised in the scope of the invention, e.g. one polyisocyanate (C) and one polyol (A); or one polyisocyanate (C) and two or more polyols (A); or two or more polyisocyanates (C) and one polyol (A); or two or more polyisocyanates (C) and two or more polyols (A). If two or more of both components is present, they do not need to be present in the same diversity, i.e. two polyisocyanates (C) are not necessarily combined with two polyols (A), but may be combined with three, four or more polyols (A), and the other way round.

Alternatively, the process according to the invention may comprise reacting a mixture comprising one or more polyisocyanates (C), one or more polyols (A) and one or more polyols (D). Once again, any combinations thereof are comprised in the scope of the invention, that is, only one of all of the components may be present; or two or more of one of the components may be present, but only one of the remaining components is present; or two or more of two of the components may be present, but only one of the remaining component is present; or two or more of all of the components is present. Again, the components do not need to be present in the same diversity, as explained above.

The process according to the invention may comprise reacting a mixture comprising one or more polyisocyanates (C), one or more compounds of formula (I) and one or more polyols (D). Again, any combinations thereof are comprised in the scope of the invention, in any diversity.

The process according to the invention may comprise reacting a mixture comprising one or more polyisocyanates (C), one or more compounds of formula (I) and one or more polyols (A). Again, any combinations thereof are comprised in the scope of the invention, in any diversity, as explained above for other possible combinations.

The process according to the invention may comprise reacting a mixture comprising one or more polyisocyanates (C), one or more compounds of formula (I), one or more polyols (A) and one or more polyols (D). Again, any combinations thereof are comprised in the scope of the invention, in any diversity, as explained above for other possible combinations.

In the process of the invention, the ratio of equivalents of polyisocyanate (C) with respect to the sum of equivalents of polyol (A) and equivalents of polyol (D) in the reaction mixture is between 2:1 and 1.2:1, preferably between 1.5:1 and 1.1:1, more preferably between 1.2:1 and 1.1:1, even more preferably between 1.1:1 and 1.05:1, most preferably 1.05:1.

In the process of the invention, the ratio between the sum of equivalents of polyol (A) and equivalents of compound of formula (I) with respect to the equivalents of polyisocyanate (C) is between 1% and 95%, preferably between 2% and 60%.

The invention is also directed to a process for obtaining polyurethanes as defined above, comprising reacting a mixture which comprises:
- a polyisocyanate (C), comprising at least two isocyanate groups;
- a compound of formula (I) as defined above; and
- a compound of formula (II) (a particular polyol (A)) as defined above;
in the presence of a catalyst,
wherein the ratio of equivalents of polyisocyanate (C) with respect to the sum of equivalents of polyol (A) and equivalents of compound of formula (I) in the reaction mixture is comprised of 2:1 and 1:1.2; preferably, between 1.5:1 and 1:1.1; more preferably between 1.2:1 and 1:1.1; still more preferably, between 1.1:1 and 1:1.05, most preferably 1.05: 1; and
wherein the ratio of the sum of equivalents of polyol (A) and equivalents of compound of formula (I) with respect to the equivalents of polyisocyanate (C) is between 1% and 95%, preferably between 2% and 60%.

The process for obtaining polyurethanes as defined above, may also comprise reacting a mixture which comprises:
- a polyisocyanate (C), selected from the group consisting of hexamethylene diisocyanate, isophorone diisocyanate, tetramethylene diisocyanate, octamethylene diisocyanate, decamethylene diisocyanate, dodecamethylene diisocyanate, and tetradecamethylene diisocyanate, preferably selected from group consisting of hexamethylene diisocyanate and isophorone diisocyanate;
- a compound of formula (I) as defined above;
- a compound of formula (II) (a particular polyol (A)) as defined above; and
- optionally, one or more polyols (D), comprising at least two hydroxyl groups, selected from the group consisting of polyester polyols, polyether polyols, aliphatic polyols, polycarbonate polyols and polyether carbonate polyols, as defined above;
in the presence of a catalyst;
wherein the ratio of equivalents of polyisocyanate (C) with respect to the sum of equivalents of polyol (A), equivalents of compound of formula (I) and equivalents of polyol (D) in the reaction mixture is comprised between 2:1 and 1:1.2, preferably between 1.5:1 and 1:1.1, more preferably between 1.2:1 and 1:1.1, even more preferably between 1.1:1 and 1:1.05, most preferably 1.05:1; and
wherein the ratio between the sum of equivalents of polyol (A) and equivalents of compound of formula (I) with respect to the equivalents of polyisocyanate (C) is between 1% and 95%, preferably between 2% and 60%.

In another aspect, the present invention relates to a polyurethane, obtainable by the processes defined above.

### Uses of polyurethane

Another aspect is related to the use of said polyurethane in the preparation of a coating, preferably incorporated as an additive of varnishes and paints, preferably in varnishes and paints for plastic and metallic substrates, more preferably in varnishes and paints for the automotive sector.

Preferably, the coating has a thickness of less than 150 µm, more preferably less than 140 µm, more preferably less than 130 µm, more preferably less than 120 µm, more preferably less than 110 µm, more preferably less than 100 µm, more preferably less than 90 µm, more preferably less than 80 µm, still more preferably less than 70 µm, still more preferably less than 60 µm, even more preferably less than 50 µm, still more preferably less than 40 µm, even more preferably less than 30 µm, most preferably less than 20 µm.

The coating agents comprising polyurethanes according to the invention are suitable for all fields of use in which coating and coating systems are used, in particular in those with high demands on the surface quality and the strength of the films, e.g. coating of surfaces of mineral construction material, varnishing and sealing of wood and wood-derived materials, coating of metal surfaces (coating of metals), coating and lacquering of asphalt or bituminous coatings, lacquering and sealing of various surfaces of plastic (coating of plastics) as well as high-gloss lacquers, in particular for the automotive sector.

The coating agents containing the polyurethanes according to the invention are usually used in monolayer lacquers or in the transparent or covering layer (upper layer) of multilayer structures.

The application of the coating may be carried out by different spraying methods, such as, for example, spraying with compressed, direct or electrostatic air, using one or possibly two spraying systems. The lacquers and coating agents containing the dispersions described above can, however, also be applied by other methods, for example by extension, rollers or blades.

### Reparation of polyurethane or a coating comprising said polyurethane

Said polyurethane as defined above or a coating comprising said polyurethane, comprises coumarin derivatives of formula (I) in its structure; normally, said coumarin derivatives may be present both in monomeric and in dimerized form. Said coumarin derivatives provide the self-repairing properties to the material, by photodimerization and photocleavage of the coumarin derivatives, and allows to obtain multiple recovery polymer systems. The more dimerized coumarin of formula (I) is contained in the polyurethane or the coating, the better mechanical properties the material will have.

"Reparation" of a polyurethane or a coating comprising it means, in the frame of the present invention, the decrease in the number or magnitude of the defects present in said coating, with "defects" being understood as the observable discontinuities when examining the coating at an increase of x20 or more. Examples of such defects are scratches and cracks. Generally, such defects have a depth of less than 70 µm, more preferably less than 65 µm, still more preferably less than 60 µm, still more preferably less than 55 µm, even more preferably less than 50 µm, even more preferably less than 45 µm, even more preferably less than 40 µm, still more preferably less than 35 µm , still more preferably less than 30 µm, even more preferably less than 25 µm, most preferred less than 20 µm.

Therefore, in another aspect, the present invention relates to a method of repairing a polyurethane or a coating comprising a polyurethane as defined above, comprising exposing said coating to light comprising a radiation with a wavelength comprised between 310 nm and 370 nm, preferably between 340 nm and 360 nm. This exposure to radiation comprising the defined wavelengths achieves the dimerization of the monomeric coumarin derivative and therefore the repair of the polyurethane. Preferably, the exposure is performed light comprising radiation of wavelengths comprised between 330 nm and 370 nm; more preferably between 340 nm and 360 nm; most preferably at 354 nm.

The repair method may comprise a prior step of exposing a polyurethane or a coating comprising the polyurethane to light comprising radiation with a wavelength comprised between 200 nm and 260 nm, which will make the repairing more effective, and make the material (i.e., the polyurethane or the coating comprising it) substantially recover the initial properties, and the resulting repaired material will be mechanically more resistant. Preferably, the irradiation is carried out at wavelengths comprised between 200 nm and 260 nm; more preferably between 240 nm and 260 nm; most preferred at 254 nm. Said irradiation cleaves the dimers that had not been cleaved by damaging the polyurethane, and subsequently the dimers are reformed by the irradiation step defined above at a higher wavelength.

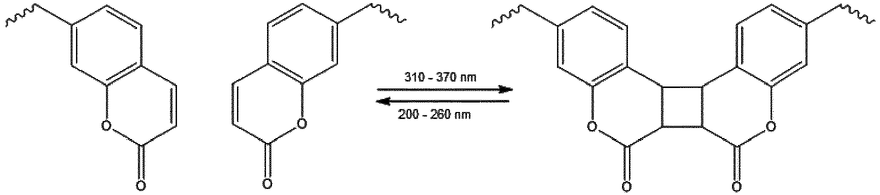

The following examples are merely illustrative and should not be considered as limiting the invention.

### EXAMPLES

### Materials and methods

Solution NMR spectra were recorded at room temperature in a Varian Unity Plus 400 instrument (Palo Alto, CA, USA) using deuterated chloroform (CDCl₃) or deuterated dimethylsulfoxide (DMSO-d6) as solvent. Spectra were referenced to the residual solvent signals at 7.26 ppm (CDCl₃) and 2.50 ppm (DMSO) for proton spectra and 77.0 ppm (CDCl₃) and 39.5 ppm (DMSO) for carbon spectra, respectively. Irradiations were carried out in a crosslinker chamber?? supplied by Ultra-Violet Products (Upland, CA, USA) equipped with two sets of 5× 8 W lamps with emission maxima at 354 nm and 254 nm. UV experiments were performed in a Perkin Elmer Lambda 35 UV/Vis spectrometer (Waltham, MA, USA). Absorbance of the thin films was measured from 450 to 210 nm. Raman spectroscopy measurements were carried out by a Renishaw inVia Laser micro-Raman Spectrometer (Wotton-under-Edge, UK). A laser beam with wavelength of 785 nm served as the excitation light. The testing area on the film was about 1 µm². The thermal transitions of the samples were analysed by DSC on a Mettler Toledo DSC 822e calorimeter (Schwerzenbach, Switzerland) equipped with a liquid nitrogen accessory. Disc samples cut from films weighing approximately 6 mg were sealed in aluminium pans. Samples were heated, from 25 °C to 120 °C at a rate of 10 °C min⁻¹, cooled to -90 °C at the maximum cooling rate of the instrument, maintained for 10 min at this temperature and re-heated from -90 °C to 120 °C at a rate of 10 °C min⁻¹. Glass transition temperatures (Tg) were taken as the midpoint of the transition. Tensile properties were measured in a MTS Synergie 200 testing machine (Eden Prairie, MN, USA) equipped with a 100 N load cell. Type 4 dumbbell test pieces (according to ISO 37) were cut from the samples. A cross-head speed of 5 mm min⁻¹ was used.

Strain was measured from cross-head separation and referred to 10 mm initial length. A minimum of 5 samples were tested for each material.

### Example 1: Synthesis of Isopropylidene-2,2-bis-(methoxy)propionic acid (DMPA)

100 g of 2,2-bis(methoxy)propionic acid (74.5 mmol) were reacted with 138 mL of 2,2-dimethoxypropane (1.12 mol) and p-toluenesulfonic acid monohydrate (7.1 g, 37.3 mmol), and then dissolved in 500 mL of acetone and introduced into a 1-neck round-bottom flask. The mixture was stirred at room temperature for 4 h. After p-toluenesulfonic acid was neutralized by adding 10 mL of NH3/ethanol (50/50, v/v) solution, the solvent was evaporated under reduced pressure at room temperature. The residue was then dissolved in Dichloromethane (DCM) and extracted three times with water. The organic phase was dried over anhydrous MgSO4 and the solvent was evaporated to produce the entitled product as white crystals.

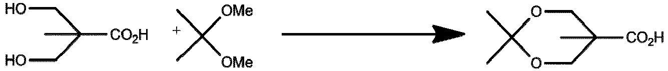

**1H RMN (400MHz, Cl₃CD, δ)**: 4.18 (d, 2H; J=12Hz), 3.68 (d, 2H; J=12Hz), 1.43 (s, 3H), 1.40 (s, 3H), 1.19 (s, 3H).

### Example 2: Synthesis of Isopropylidene-2,2-bis-(methoxy)propionic acid anhydride (DMPAA)

Isopropylidene-2,2-bis(methoxy)propionic acid (30 g, 90.8 mmol) was dissolved in 50 mL of anhydrous DCM. N,N-Dicyclohexylcarbodiimide (DCC) (19.74 g, 94.7 mmol) was added to the mixture, and the esterification reaction was continued at room temperature for 12 h under N₂. The N,N-dicyclohexylurea (DCU) by product was filtered off three times as the solvent was condensed and finally evaporated. The residue was further dried under vacuum to obtain isopropylidene-2,2-bis-(methoxy)propionic acid anhydride as a viscous oil.

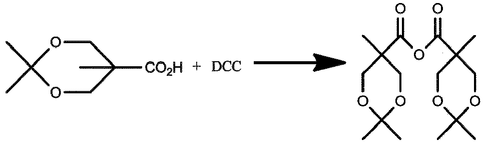

**1H RMN (400MHz, Cl₃CD, δ)**: 4.19 (d, 2H; J=12Hz), 3.66 (d, 2H; J=12Hz), 1.42 (s, 3H), 1.37 (s, 3H), 1.22 (s, 3H).

### Example 3: Synthesis of 3-methoxy-2-(methoxymethyl)-2-methyl-N-(4-methyl-2-oxo-2H-chromen-7-yl)propanamide

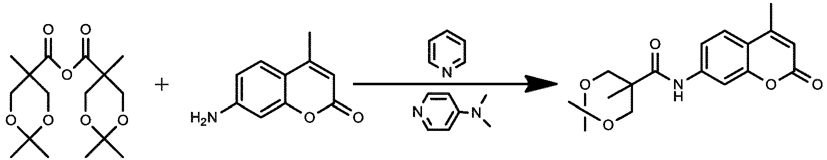

The previously synthesized isopropylidene- 2.2-bis-(methoxy)propionic anhydride (15.9g, 48.4mmol)(DMPAA) was dissolved in 10 mL anhydrous pyridine and 30mL anhydrous dimethylacetamide. Afterwards, 7-amino-4-methyl-2H-chromen-2-one (5 g, 28.5 mmol) and N,N-dimethylpyridin-4-amine were added to the system which was subsequently warmed up to 40°C and stirred for 48h. Resulting solid was precipitated over 200 mL water and 2 mL HCI to obtain the final product. Obtained crystals were further dried under vacuum to obtain 3-methoxy-2-(methoxymethyl)-2-methyl-N-(4-methyl-2-oxo-2H-chromen-7-yl)propanamide.
**1H RMN (400MHz, Cl₃CD, δ)**: 9.68 (s, 1H), 7.82 (d, J = 1.9 Hz, 1H), 7.72 (d, J = 8.7 Hz, 1H), 7.58 (dd, J = 8.7, 1.9 Hz, 1H), 6.26 (s, 2H), 4.16 (d, J = 11.9 Hz, 2H), 3.75 (d, J = 11.9 Hz, 2H), 2.40 (s, 5H), 1.40 (s, 3H), 1.31 (s, 3H), 1.18 (s, 3H).

### Example 4: Synthesis of 3-hydroxy-2-(hydroxymethyl)-2-methyl-N-(4-methyl-2-oxo-2H-chromen-7-yl)propanamide

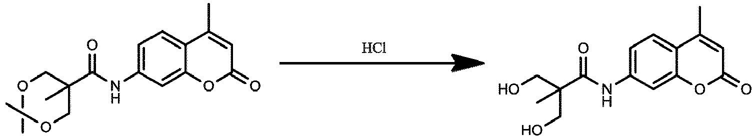

The obtained product (3-methoxy-2-(methoxymethyl)-2-methyl-N-(4-methyl-2-oxo-2H-chromen-7-yl)propanamide) was dissolved in 100 mL of tetrahydrofuran. Afterwards, 40 mL of hydrochloric acid (2.4 mol/L) were dropwise added to the system and stirred overnight at room temperature. The solvent was removed under vacuum and the resulting solid was then washed in H2O. The 3-hydroxy-2-(hydroxymethyl)-2-methyl-N-(4-methyl-2-oxo-2H-chromen-7-yl)propanamide product was dried in a vacuum oven.
**¹H NMR (300 MHz, DMSO, δ6)** 9.65 (s, 1H), 7.81 (d, 1H; J1=2.1Hz), 7.67 (d, 1H, J=8.7Hz), 7.54 (dd, 1H, J₁=2.1Hz, J₂=6.6 Hz), 6.22 (s, 1H), 4.45 (s, 3H), 3.62 (dd, 4H, J₁=10.8 Hz, J₂=7.5 Hz), 3.08 (s, 3H), 1.14 (s, 3H), 1.41 (s, 3H), 1.35 (s, 3H), 1.18 (s, 3H).

### Example 5: Synthesis of coumarin-polycaprolactone diols

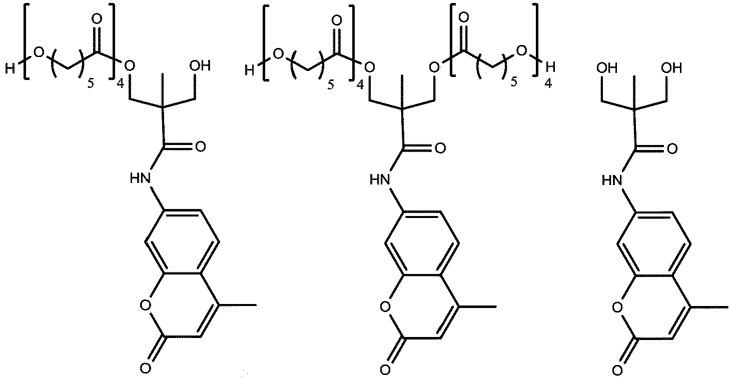

Polymerization was carried out following a general procedure for the ring opening polymerization of ε-caprolactone. As an example, the synthesis of the coumarin-containing polycaprolactone diol of approximately 1200 g·mol⁻¹ molecular weight based on 3-hydroxy-2-(hydroxymethyl)-2-methyl-N-(4-methyl-2-oxo-2H-chromen-7-yl)propanamide is described. In a 100 mL round-bottomed flask previously dried, ε-caprolactone (23.4g 205mmol),3-hydroxy-2-(hydroxymethyl)-2-methyl-N-(4-methyl-2-oxo-2H-chromen-7-yl) propanamide (6.8g, 23.3mmol) and SnOct₂ (0.1% weight respect to the monomer weight) were charged and heated with magnetic stirring at 120 °C for 24 h. When hot, a vacuum pump was connected (final pressure approximately 100 mbar) to remove unreacted ε-caprolactone for 2 h. Pump was disconnected and the product let to cool at room temperature. A solid was obtained with almost quantitative yield. The molecular weight of this coumarin-containing polycaprolactone diol (PCLcoum) was calculated by 1H-NMR and a value of 1191 g·mol⁻¹ was obtained.
**¹H NMR (300 MHz, DMSO, δ6)** 6.19 (d, 2H, J=5.7Hz), 4.52 (d, 1H, J₁=9.0Hz), 4.33 (s, 3H), 4.45 (s, 3H), 4.01 (m, 38H), 3.76 (s, 2H), 3.60 (t, 6H, J=4.8Hz).

### Example 6: Synthesis of coumarin-polycaprolactone polyurethanes, and preparation of a film (coating) therewith

The synthesis of all polyurethanes was carried out using dimethylacetamide as solvent, with approximately a 5% molar excess of diisocyanate respect to the total molar content of diols in the reaction.

Two type of polyurethanes derived from 3-hydroxy-2-(hydroxymethyl)-2-methyl-N-(4-methyl-2-oxo-2H-chromen-7-yl)propanamide were synthesized by reacting PCL527, IPDI, BD and coumarin derivative: a linear segmented polyurethane with coumarin derivative within the soft segment, and a linear segmented polyurethane with coumarin derivative within the hard segment.

In the case of the linear segmented polyurethane with coumarin derivative within the hard segment, in a 25mL round-bottom flask, 1.50g (2.87mmol) of PCL527, 0.13g (0.43 mmol) of 3-hydroxy-2-(hydroxymethyl)-2-methyl-N-(4-methyl-2-oxo-2H-chromen-7-yl)propanamide, 0.04 g (0.45 mmol) of BD and 0.83 g (3.7 mmol) of IPDI were dissolved in 8 mL of dimethylacetamide. Three drops of SnOct₂ catalyst were added and the stirred solution heated at 80 °C for 3 h, followed by 24 h stirring at ambient temperature. The resulting polymeric solution was casted into a levelled glass and the solvent evaporated at room temperature for 48 h. Polymeric films of different thickness were prepared from this material.

In the case of the linear segmented polyurethane with coumarin derivative within the soft segment, in a 25 mL round-bottom flask, 0.99 g (1.89 mmol) of PCL527, 0.51g (0.43 mmol) of synthesized PCL based on 3-hydroxy-2-(hydroxymethyl)-2-methyl-N-(4-methyl-2-oxo-2H-chromen-7-yl)propanamide, 0.14 g (1.55 mmol) of BD and 0.86 g (3.87 mmol) of IPDI were dissolved in 11 mL of dimethylacetamide. Three drops of SnOct₂ catalyst were added and the stirred solution heated at 80 °C for 3 h, followed by 24 h stirring at ambient temperature. The resulting polymeric solution was casted into a levelled glass and the solvent evaporated at room temperature for 48 h. Polymeric films of different thickness were prepared from this material.

### Example 7: Mechanical resistance to traction of polyurethanes obtained from cumarin derivatives

The polyurethane obtained according to Example 6 was submitted to testing, to determine its mechanical resistance to traction, in order to be compared to the cumarin derivatives disclosed in prior art document WO2017/103295.

In the testing, the polyurethane of WO2017/103295 showed a mechanical resistance to traction of 0.6MPa breaking strength before irradiation at wavelengths between 300 and 370, which was increased up to 52 MPa once the polyurethane was submitted to such irradiation.

The polyurethane obtained in Example 6, (i.e., the linear segmented polyurethane with coumarin derivative within the soft segment) however, already showed a mechanical resistance to traction of about 37MPa breaking strength before irradiation, which is almost one magnitude higher than the prior art material.

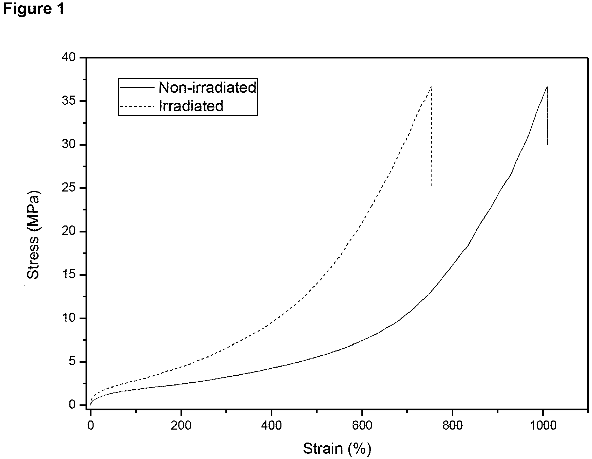

The results obtained are shown in Fig. 1, wherein the stress (MPa) versus the percentage of strain is represented.

## Claims

**1.** Compound of formula (I): 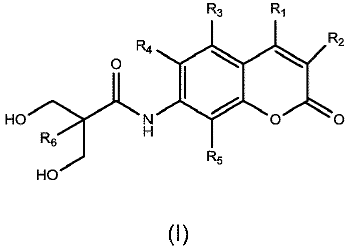 wherein
R₁, R₂, R₃, R₄, R₅ and R₆ are independently selected from the group consisting of H, C₁-C₆ alkyl and C₁-C₆ alkoxyl; or a stereoisomer thereof.

**2.** Compound of formula (I) according to claim 1, wherein
R₁ and R₆ are independently selected from the group consisting of H, methyl and ethyl; and
R₂, R₃, R₄ and R₅ are H.

**3.** Compound of formula (I) according to any one of the preceding claims, wherein
R₁ and R₆ are methyl; and
R₂, R₃, R₄ and R₅ are H.

**4.** Polyol (A), obtainable by reaction of one or more compounds of formula (I) as defined in any one of claims 1 to 3 with one or more compounds (B), in presence of a catalyst, wherein the compound (B) comprises:
a) a functional group selected from -C(=O)-O- and -O- and optionally at least one hydroxyl group, with the proviso that, when the functional group selected from - C(=O)-O- and -O- is not part of a cycle, at least one hydroxyl group must be present; or
b) a functional group -O-CH(CH₃)-(C=O)-, and at least one hydroxyl group.

**6.** Polyol (A) according to any one of claims 4 or 5, wherein compound (B) is a lactone.

**7.** Polyol (A) according to any one of claims 4 or 6, which is a compound of formula (II): 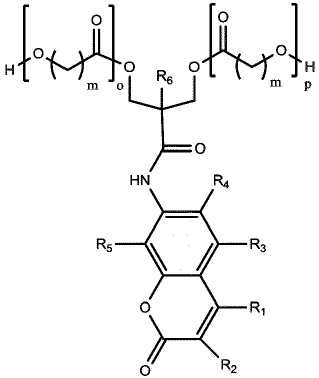 wherein
R₁, R₂, R₃, R₄, R₅ and R₆ are as defined in any one of claims 1 to 3, and
**o** and **p** are independently selected from an integer from 0 to 40, **m** is an integer from 2 to 10, with the proviso that at least one of **o** and **p** is different from zero.

**8.** Process for obtaining a polyurethane, comprising reacting a mixture that comprises:
- one or more polyisocyanates (C), which comprise at least two isocyanate groups;
- one or more polyols, independently selected from one or more polyols (A) as defined in any one of claims 4 to 7 and one or more compounds of formula (I) as defined in any one of claims 1 to 3, or mixtures thereof; and
- optionally, one or more polyols (D), which comprise at least two hydroxyl groups, selected from the group consisting of polyester polyols, polyether polyols, aliphatic polyols, polycarbonate polyols, polyether carbonate polyols and polyols of natural origin;
in an aprotic organic solvent;
in the presence of a catalyst;
wherein the ratio of equivalents of polyisocyanate (C) with respect to the sum of equivalents of polyol (A), equivalents of compound of formula (I) and equivalents of optional polyol (D) in the reaction mixture is comprised between 2:1 and 1:1.2; and wherein the ratio of the sum of equivalents of polyol (A) and equivalents of compound of formula (I) with respect to the equivalents of polyisocyanate (C) is comprised between 1% and 95%.

**9.** Process for obtaining a polyurethane according to claim 8, comprising reacting a mixture that comprises:
- one or more polyisocyanates (C), comprising at least two isocyanate groups;
- one or more compounds of formula (I) as defined in any one of claims 1 to 3; and
- one or more polyols (D), comprising at least two hydroxyl groups, selected from the group consisting of polyester polyols, polyether polyols, allyl polyols, polycarbonate polyols, polyether carbonate polyols and polyols of natural origin;
in an aprotic organic solvent;
in the presence of a catalyst;
wherein the ratio of the equivalents of polyisocyanate (C) with respect to the sum of equivalents of polyol (D) and equivalents of compound of formula (I) in the reaction mixture is between 2:1 and 1:1.2; and
wherein the ratio between the sum of equivalents of polyol (D) and equivalents of compound of the formula (I) with respect to the equivalents of polyisocyanate (C) is comprised between 1% and 95%.

**10.** The process according to any one of claims 8 or 9, wherein
• the polyisocyanate (C) is selected from the group consisting of linear or branched C₂-C₂₀ alkylene diisocyanate, optionally substituted with 1 to 10 substituents, independently selected from halogens; C₃-C₈ cycloalkylene diisocyanate, optionally substituted with 1 to 4 substituents, independently selected from the group consisting of C₁-C₃ alkyl and halogen; C₁-C₆-alkylene-C₃-C₈ cycloalkylene diisocyanate, optionally substituted with 1 to 4 substituents, independently selected from the group consisting of C₁-C₃ alkyl and halogen; and C₃-C₈-cycloalkylene - C₁-C₆-alkylene - C₃-C₈-cycloalkylene diisocyanate, optionally substituted with 1 to 4 substituents, independently selected from the group consisting of C₁-C₃ alkyl and halogen; and/or
• the polyol (D) is selected from the group consisting of polycaprolactones, polyethylene glycols, polypropylene glycols, mixed polyglycols of ethylene oxide and propylene oxide, polytetramethylene glycols, and polytetrahydrofurans, and the polyol (D) has an average molecular weight between 200 Dalton and 10000 Dalton; and/or
• the catalyst is selected from the group consisting of tin octanoate, tin isooctanoate, dibutyltin dilaurate, triethylamine, triethylene diamine, methylethanolamine, triethanolamine, dimethylethanolamine, pyridine, 1,4-diazabicyclo [2.2.2] octane, dimethylcyclohexylamine and potassium octanoate; and/or
• the aprotic organic solvent is selected from the group consisting of dimethylformamide, butyl acetate and mixture thereof.

**11.** The process according to any one of claims 8 to 10, comprising reacting a mixture that comprises:
- a polyisocyanate (C), selected from the group consisting of hexamethylene diisocyanate, isophorone diisocyanate, tetramethylene diisocyanate, octamethylene diisocyanate, decamethylene diisocyanate, dodecamethylene diisocyanate, and tetradecamethylene diisocyanate;
- one or more compounds of formula (I) as defined in any one of claims 1 to 3; and
- one or more polyols (A) as defined in claim 7; and
- optionally, one or more polyols (D), comprising at least two hydroxyl groups, selected from the group consisting of polyester polyols, polyether polyols, aliphatic polyols, polycarbonate polyols, polyether carbonate polyols and polyols of natural origin;
in the presence of a catalyst;
wherein the ratio of the equivalents of polyisocyanate (C) with respect to the sum of equivalents of polyol (A), equivalents of optional polyol (D) and equivalents of compound of formula (I) in the reaction mixture is comprised between 2:1 and 1:1.2, and
wherein the ratio of the sum of equivalents of polyol (A) and equivalents of compound of the formula (I) with respect to the equivalents of polyisocyanate (C) is comprised between 1% and 95%.

**12.** Polyurethane, obtainable by the process defined in any one of claims 8 to 11.

**13.** Use of a polyurethane according to claim 12, in the preparation of a coating.

**14.** Process for repairing a polyurethane as defined in claim 12 or a coating that comprises said polyurethane, the process comprising the steps of:
a) optionally, exposing said polyurethane or said coating to light comprising radiation with a wavelength comprised between 200 and 260 nm;
b) exposing said polyurethane or said coating to light comprising radiation with a wavelength comprised between 310 nm and 370 nm.

**15.** A process for obtaining a compound of formula (I), comprising an amidation reaction between an acid (IV), or a precursor thereof, selected from a corresponding anhydride or acid halide, and coumarin (III): 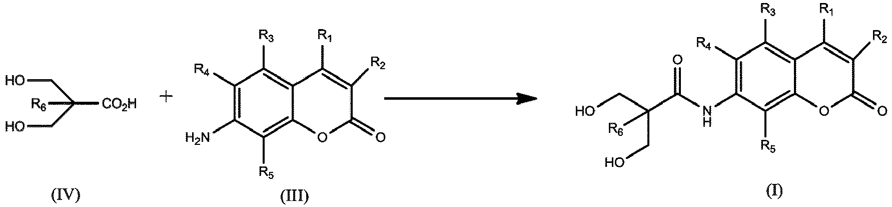 wherein R₁, R₂, R₃, R₄, R₅ and R₆ have the meanings defined above for compound of formula (I).
